**Europäisches Patentamt**

**European Patent Office**

**Office européen des brevets**

(19)

(11) Veröffentlichungsnummer : **0 159 007**
**B1**

# EUROPÄISCHE PATENTSCHRIFT

(12)

(45) Veröffentlichungstag der Patentschrift :
**11.01.89**

(21) Anmeldenummer : **85104558.3**

(22) Anmeldetag : **15.04.85**

(51) Int. Cl.⁴ : **A 61 F 5/00**, A 61 B 17/00

(54) **Vorrichtung zum Einrichten einer Wirbelsäule.**

(30) Priorität : **16.04.84 DE 3414374**

(43) Veröffentlichungstag der Anmeldung :
**23.10.85 Patentblatt 85/43**

(45) Bekanntmachung des Hinweises auf die Patenterteilung : **11.01.89 Patentblatt 89/02**

(84) Benannte Vertragsstaaten :
**CH FR GB IT LI NL**

(56) Entgegenhaltungen :
**DE--A-- 3 219 575**
**PL--A-- 96 695**
**US--A-- 2 774 350**
**US--A-- 4 386 603**

(73) Patentinhaber : **Kluger, Patrick, Dr. med.**
**Heinrich-Hammer-Strasse 12**
**D-7904 Erbach (DE)**

(72) Erfinder : **Kluger, Patrick, Dr. med.**
**Heinrich-Hammer-Strasse 12**
**D-7904 Erbach (DE)**

(74) Vertreter : **Walther, Horst, Dipl.-Ing.**
**Wilhelmshöher Allee 275 Postfach 41 01 08**
**D-3500 Kassel (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung zum Einrichten einer Wirbelsäule mit geschädigten Wirbelkörpern, wobei Tragbolzen in die Wirbelkörper beiderseits des geschädigten Wirbelkörpers eingesetzt sind, um ein Stützsystem anzubringen.

Bekanntlich muß einer Wirbelsäule mit beschädigten Wirbelkörpern dadurch wieder zur Stabilität verholfen werden, daß ein Stützsystem in die Wirbelkörper beiderseits des geschädigten Wirbelkörpers eingesetzt wird. Dabei muß zunächst ein Einrichten und daraufhin das Stabilisieren der Wirbelsäule erfolgen. Es ist schon eine Vorrichtung gemäß DE-A-3 219 575, die auf den selben Erfinder zurückgeht wie der Gegenstand der vorliegenden Anmeldung, bekannt. Das Stabilisieren der Wirbelsäule erfolgt dabei in der Weise, daß der geschädigte Wirbelkörper mechanisch überbrückt wird. Hierzu dienen Spannschrauben, die die übereinanderstehenden Tragbolzen, die in die gesunden Wirbelkörper eingesetzt sind, miteinander verbinden und dadurch die Kraftübertragung übernehmen. Die Spannschrauben bestehen aus einer Gewindehülse, in deren Enden Schraubbolzen mit gegensinnigen Gewinde eingeschraubt sind. Dadurch kann die die Tragbolzen verbindende Spannschraube durch Drehen der Gewindehülse zum Einrichten und zum Stabilisieren verwendet werden.

Da zum Einrichten der Wirbelsäule wegen der erforderlichen Hebelkräfte relativ lange Tragbolzen benötigt werden, muß nach erfolgter Stabilisierung der Wirbelsäule ein Abschneiden der überstehenden Längen der Tragbolzen erfolgen, damit die Implantation des gesamten Stützsystems erfolgen kann. Das bringt jedoch Erschütterungen dieses Systems mit sich ; außerdem ist unvermeidlich, daß Metallstaub in die Wunde gelangt. Hinzukommt, daß das Stützsystem eine große Materialmenge darstellt, die implantiert werden muß. Außerdem ist die Operation wegen der Vielzahl der in der Tiefe der Wunde einzusetzenden Schrauben technisch schwierig und erhöht die Operationsdauer.

In bezug auf das bloße Einrichten einer Wirbelsäule ist eine Vorrichtung nach der polnischen Druckschrift PL-A-96 695 bekannt, wobei es dort nur darum geht bei einer an sich gesunden, aber gekrümmten Wirbelsäule die Krümmung durch einen Distraktionsstab nach und nach zu beseitigen, indem verstellbare Arme, die über entsprechenden Zapfen und Hackenhalter auf in die Wirbelsäule eingesetzte Hacken wirken, mit Hilfe eines Zahnradgetriebes verstellt, und zwar auseinandergezogen werden, damit die Krümmung beseitigt wird. Diese Vorrichtung reicht aber wegen ihrer begrenzten Bewegungsfreiheit für die Belange des Ausrichtens einer durch Verletzung fehlstehenden Wirbelsäule nicht aus.

Der Erfindung liegt daher die Aufgabe zugrunde, das Einrichten der Wirbelsäule mit anderen Mitteln vorzunehmen, als denen, die zur Stabilisierung dienen.

Nach der Erfindung besteht die Vorrichtung aus einem ersten auf einer Führungsachse verstellbaren und einen zweiten auf der Führungsachse ortsfesten Arm, und ist dadurch gekennzeichnet, daß jeder Arm endseitig eine um zwei senkrecht zueinander stehende Achsen frei verstellbare Aufnahmehülse trägt, die je eine Verlängerungsstange aufnimmt, die auf den Tragbolzen abnehmbar aufgesetzt ist, der in den Wirbelkörper angebracht ist, und daß jeder Tragbolzen eine Befestigungsfläche für einen zwischen den Tragbolzen einspannbaren Fixierstab, z. B. eine Spannschraube, aufweist.

Im einzelnen ist die Ausbildung dabei so getroffen, daß die Aufnahmehülse eine Ansatzfläche mit Rasterung trägt, die auf einer Stützfläche mit Rasterung lösbar befestigt ist, wobei die Stützfläche gelenkig und feststellbar am Arm angebracht ist. Dadurch kann zunächst die Stützfläche um die Gelenkachse verstellt werden, außerdem kann auch die Ansatzfläche mit ihrer Rasterung auf der Stützfläche selbst verstellt werden. Die Befestigung der Ansatzfläche mit der Stützfläche erfolgt dabei im allgemeinen durch eine Schraubverbindung, wobei dann in der Ansatzfläche eine entsprechende Bohrung angebracht sein muß.

Die Führungsachse, auf der die beiden Arme angebracht sind, trägt ein Gewinde, das eine Stellmutter aufnimmt, die an dem verstellbaren Arm angreift.

Sind die Verlängerungsstangen auf die Tragbolzen aufgesetzt, dann kann das Einrichten der Wirbelsäule dadurch erfolgen, daß die Vorrichtung mit Hilfe der Aufnahmehülsen auf die Verlängerungsstangen aufgeschoben und dort befestigt wird. Durch Drehen an der Stellmutter werden die beiden Arme aufeinanderzu- oder wegbewegt, so daß auf die Tragbolzen ein entsprechendes Biegemoment ausgeübt wird, das zu einer Einrichtung bzw. Ausrichtung der Wirbelsäule führt.

Der Tragbolzen trägt koaxial ein Gewinde zur Befestigung der Verlängerungsstange. Ferner trägt der Tragbolzen eine Befestigungsfläche für eine Spannschraube.

Ist das Einrichten der Wirbelsäule erfolgt, dann kann durch Anbringen einer Spannschraube oder eines Fixierstabes zwischen den mit Rasterung versehenen Befestigungsfläche der beiden Tragbolzen die Stabilisierung der Wirbelsäule erfolgen. Da die Verlängerungsstangen abnehmbar sind, kann mithin der Tragbolzen relativ kurz ausgebildet werden, da er nämlich nicht zum Einrichten der Wirbelsäule benötigt wird, sondern lediglich zur Stabilisierung. Es kann dann auch eine Spannschraube verwendet werden, die nicht aus einer Gewindehülse mit zwei endseitig eingesetzten Schraubbolzen besteht, sondern lediglich eine Spannschraube aus Spannhülse und Schraubbolzen, die jeweils endseitig eine Befestigungsfläche mit Rasterung aufweisen, um an die Befestigungsflächen der Tragbolzen angesetzt zu werden.

Damit wird deutlich, daß zum Einrichten der Wirbelsäule eine besondere Vorrichtung verwendet wird, die nach erfolgter Einrichtung abgenommen werden kann, wobei ein Absägen der Tragbolzen nicht erforderlich ist, da die Verlängerungsstangen abnehmbar an den relativ kurzen Tragbolzen angebracht sind.

Die Arme sind winkelförmig und räumlich gekrümmt ausgebildet. Dadurch ist das Operationsfeld leichter zugänglich, weil sich die Arme von der Wunde weg erstrecken.

In der Zeichnung ist eine beispielsweise Ausführungsform dargestellt.

Fig. 1 zeigt die erfindungsgemäße Vorrichtung in Ansicht ;

Fig. 2 zeigt schematisch die Anordnung der Vorrichtung an den Tragbolzen, die in den Wirbelkörpern angebracht sind ;

Fig. 3 zeigt einen Schnitt gemäß der Linie III-III.

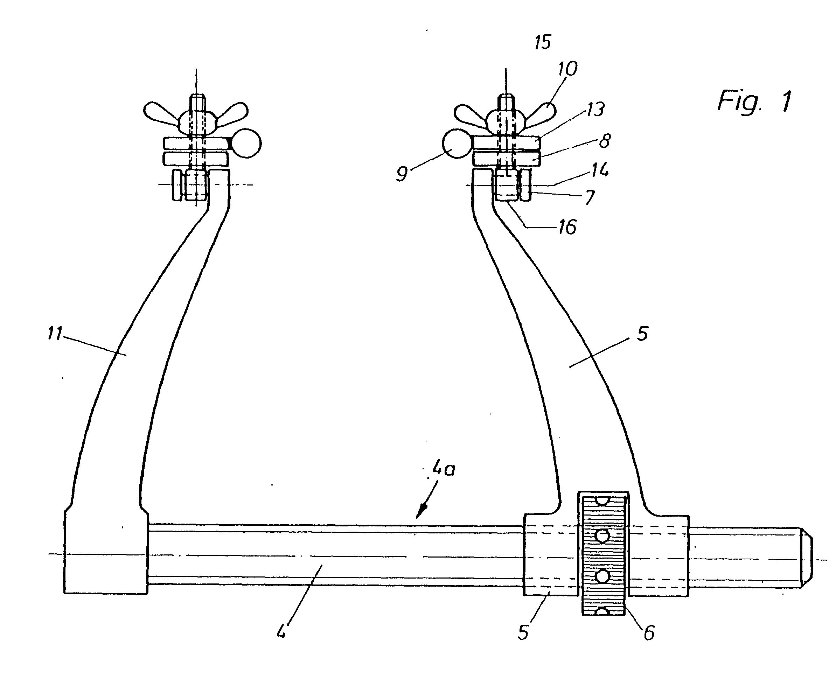

Wie Fig. 1 zeigt, besteht die Vorrichtung aus einer Führungsachse 4, auf der der Arm 5 verstellbar und der Arm 11 ortsfest angebracht sind. Zum Zwecke der Verstellung ist die Führungsachse 4 mit dem Gewinde 4a versehen, auf der eine Stellmutter 6 angebracht ist, die an dem Arm 5 angreift. Dadurch kann der Abstand der Arme 5 bzw. 11 durch Drehen an der Stellmutter 6 verändert werden. Am Ende der Arme ist je eine Aufnahmehülse 9 um zwei Achsen verstellbar angebracht. Die Ausbildung ist dabei so getroffen, daß die Aufnahmehülse 9 eine Ansatzfläche 13 mit Rasterung aufweist, die auf der Stützfläche 8 lösbar mittels der Schraubverbindung 10 befestigt ist. Die Stützfläche 8 besitzt dabei eine Buchse 16, die auf einem entsprechenden Zapfen, der am Armende angebracht ist, gelenkig angeordnet ist. Das Feststellen der Hülse 16 geschieht mit Hilfe einer Schraube 14 oder dergleichen, sofern es erforderlich sein sollte. Somit kann die Aufnahmehülse 9 um die Achse 15 und außerdem noch um die Achse 14 geschwenkt werden. Die Ausbildung an dem Arm 11 ist die gleiche.

Der Arm 5 bzw. 11 ist etwa winkelförmig ausgebildet und außerdem noch räumlich gekrümmt, damit das Operationsfeld möglichst leicht zugänglich ist.

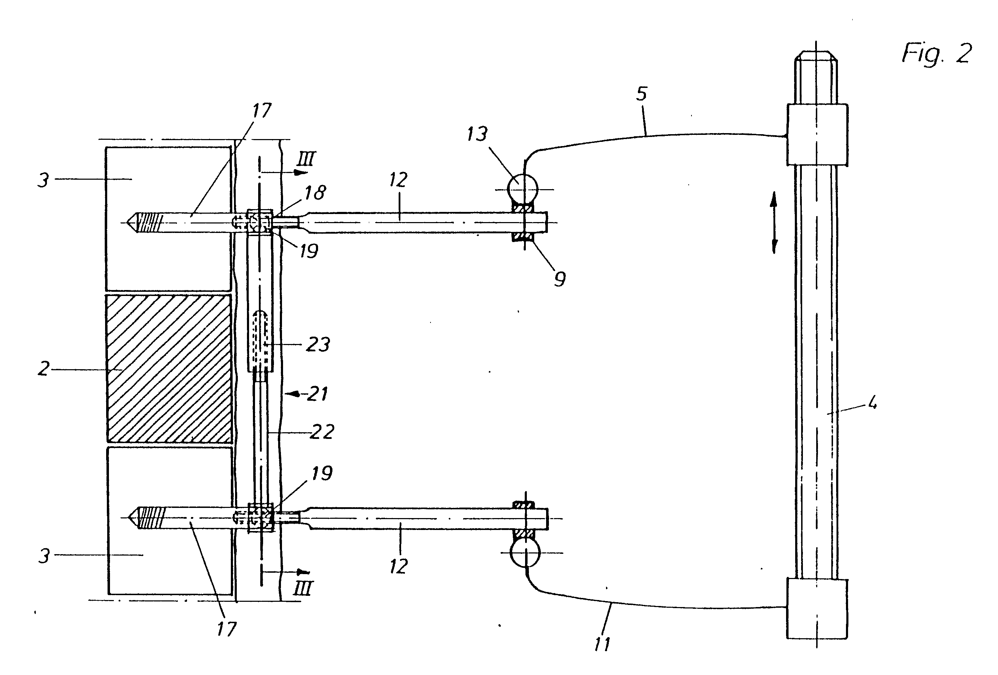

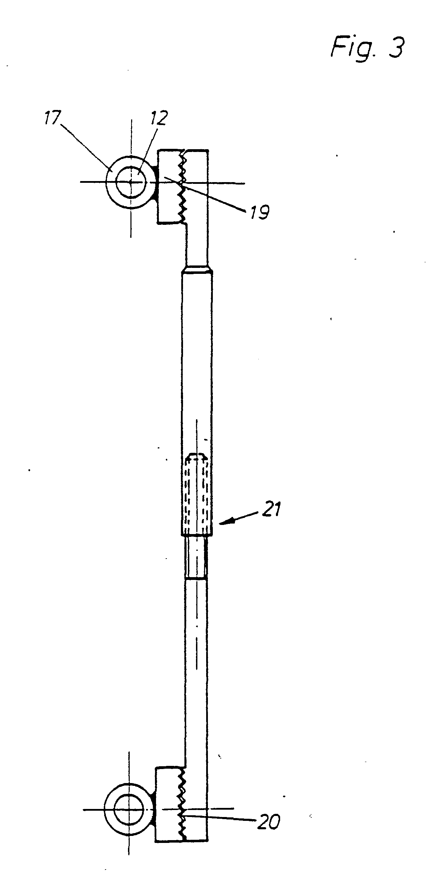

Die Fig. 2 zeigt die Handhabung der Vorrichtung. In die gesunden Wirbelkörper 3 ist in an sich bekannter Weise der Tragbolzen 17 eingeschraubt. Er besitzt am Ende eine Vertiefung 18 mit Innengewinde, damit die Verlängerungsstange 12 darin eingeschraubt werden kann. Das Gewinde liegt mithin koaxial zum Tragbolzen 17. Seitlich von dem Tragbolzen 17 ist eine Befestigungsfläche 19 mit einer Rasterung 20 angebracht, damit die beiden Tragbolzen zum Zwecke der Stabilisierung der Wirbelsäule durch eine Spannschraube 21 miteinander verbunden werden können. Diese Spannschraube besteht aus einem Gewindebolzen 22 und einer Gewindehülse 23 (Fig. 3).

Ist mit Hilfe der Vorrichtung die Wirbelsäule eingerichtet, dann erfolgt die Stabilisierung mit Hilfe der Spannschraube 21. Als dann können die Verlängerungsstangen 12 abgenommen werden. Da mithin relativ kurze Tragbolzen zur Anwendung kommen können, ist ein nachträgliches Absägen dieser Bolzen nicht erforderlich, denn sie sind leicht in die Muskulatur implantierbar.

**Patentansprüche**

1. Vorrichtung zum Einrichten einer Wirbelsäule mit geschädigten Wirbelkörpern, bei der Tragbolzen in die Wirbelkörper beiderseits des geschädigten Wirbelkörpers einsetzbar sind, bestehend aus einem ersten auf einer Führungsachse (4) verstellbaren (5) und einem zweiten auf der Führungsachse ortsfesten Arm (11), dadurch gekennzeichnet, daß jeder Arm (5, 11) endseitig eine um zwei senkrecht zueinander stehende Achsen frei verstellbare Aufnahmehülse (9) trägt, wobei jede Aufnahmehülse (9) eine Verlängerungsstange (12) aufnimmt, die auf den Tragbolzen abnehmbar aufgesetzt ist, der in den Wirbelkörper einsetzbar ist, und daß jeder Tragbolzen eine Befestigungsfläche (19) für einen zwischen den Tragbolzen einspannbaren Fixierstab, z. B. eine Spannschraube (21), aufweist.

2. Vorrichtung nach Anspruch 1 dadurch gekennzeichnet, daß die Aufnahmehülse (9) eine Ansatzfläche (13) mit Rasterung trägt, die auf einer Stützfläche (8) mit Rasterung lösbar befestigt ist, wobei die Stützfläche (8) gelenkig am Arm (5, 11) angebracht ist.

3. Vorrichtung nach Anspruch 1 dadurch gekennzeichnet, daß die Führungsachse (4) ein Gewinde trägt, das eine Stellmutter (6) aufnimmt, die am verstellbaren Arm (5) angreift.

4. Vorrichtung nach Anspruch 1 dadurch gekennzeichnet, daß der Arm (5, 11) winkelförmig und räumlich gekrümmt ist.

5. Vorrichtung nach Anspruch 1 dadurch gekennzeichnet, daß der Tragbolzen (17) koaxial ein Gewinde oder ein ähnliches Aufnahmeteil zur Befestigung der Verlängerungsstange (12) trägt.

**Claims**

1. Device for setting a vertebral column with damaged vertebrae, in which device carrier pins are insertable into the vertebrae at both sides of the damaged vertebra and which consists of a first arm (5) displaceable on a guide axle (4) and a second arm (11) fixedly located on the guide axle, characterised thereby, that each arm (5, 11) at the end carries a receptacle sleeve (9), which is freely displaceable about two axes each standing perpendicularly to the other, wherein each receptacle sleeve (9) receives an extension rod (12), which is removably placed on the carrier pin which is insertable into the vertebra, and that each carrier pin displays a fastening surface (19) for a fixing rod, for example a tightening screw (21), tightenable between the carrier pins.

2. Device according to claim 1, characterised thereby, that the receptable sleeve (9) carries an attachment surface (13) with grating, which is detachably fastened on a support surface (8) with grating, wherein the support surface (8) is articulatedly mounted at the arm (5, 11).

3. Device according to claim 1, characterised thereby, that the guide axle (4) carries a thread which receives a setting nut (6), which engages at the displaceable arm (5).

4. Device according to claim 1, characterised thereby, that the arm (5, 11) is curved angularly and in three dimensions.

5. Device according to claim 1, characterised thereby, that the pin (17) co-axially carries a thread or a similar receptacle part for the fastening of the extension rod (12).

**Revendications**

1. Dispositif pour ajuster une colonne vertébrale ayant des vertèbres endommagées, comportant des chevilles de support agencées pour être fixées dans les vertèbres situées de part et d'autre de la vertèbre endommagée, un premier bras (5) réglable en position sur un axe de guidage (4), et un second bras (11) en position fixe sur l'axe de guidage, caractérisé en ce que chaque bras (5, 11) porte à une extrémité une douille de maintien (9) qui est réglable librement autour de deux axes orthogonaux entre eux, chaque douille (9) maintenant une tige de rallonge (12) montée de manière amovible sur la cheville de support agencée pour être fixée dans ladite vertèbre, et en ce que chaque cheville de support comporte une surface de fixation (19) pour une barre de fixation agencée pour relier lesdites chevilles, notamment une barre à vis (21).

2. Dispositif selon la revendication 1, caractérisé en ce que la douille de maintien (9) comporte une surface d'épaulement (13) pourvue de crans et fixée de manière amovible sur une surface d'appui (8) pourvue de crans, ladite surface d'appui (8) étant montée de manière articulée sur le bras (5, 11).

3. Dispositif selon la revendication 1, caractérisé en ce que l'axe de guidage (4) comporte un filetage sur lequel est engagé un écrou de réglage (6) qui est en prise sur le bras réglable (5).

4. Dispositif selon la revendication 1, caractérisé en ce que le bras (5, 11) est coudé et courbe dans l'espace.

5. Dispositif selon la revendication 1, caractérisé en ce que la cheville de support (17) comporte, coaxialement à elle-même, un filetage ou un organe de montage similaire pour la fixation de la tige de rallonge (12).

Fig. 1

15
10
13
8
14
7
16
9
5
6
5
4a
4
11

1

Fig. 2

EP 0 159 007 B1

*Fig. 3*